# EUROPEAN PATENT SPECIFICATION

(11) **EP 3 431 580 B1**
(45) Date of publication and mention of the grant of the patent: **12.01.2022**
(21) Application number: 17766484.4
(22) Date of filing: 08.03.2017
(51) Int. Cl.: C12N 1/20, A23K 30/15, A61K 35/74, A61K 35/747, A61P 43/00

(54) **LACTIC ACID BACTERIA FOR SILAGE PREPARATION AND ADDITIVE FOR SILAGE PREPARATION**
MILCHSÄUREBAKTERIEN ZUR HERSTELLUNG VON SILAGE UND ADDITIV ZUR HERSTELLUNG VON SILAGE
BACTÉRIES LACTIQUES DESTINÉES À LA PRÉPARATION D'ENSILAGE ET ADDITIF DESTINÉ À LA PRÉPARATION D'ENSILAGE

(30) Priority: 15.03.2016 JP 2016050808
(43) Date of publication of application: 23.01.2019
(73) Proprietor: Snow Brand Seed Co., Ltd., Hokkaido 004-8531 (JP)
(72) Inventor: HONMA, Mitsuru, Ebetsu-shi Hokkaido 069-0832 (JP); KITAMURA, Toru, Ebetsu-shi Hokkaido 069-0832 (JP)
(74) Representative: Grünecker Patent- und Rechtsanwälte PartG mbB
(86) International application number: PCT/JP2017/009185
(87) International publication number: WO 2017/159483

(56) References cited:
- WO-A1-2016/030456
- WO-A2-2007/103032
- WO-A2-2007/103032
- JP-A- 2009 044 971
- US-A1- 2005 281 917
- US-A1- 2005 281 917
- KROONEMAN J ET AL: "Lactobacillus diolivorans sp. nov., a 1,2-propanediol-degrading bacterium isolated from aerobically stable maize silage", INTERNATIONAL JOURNAL OF SYSTEMATIC AND EVOLUTIONARY MICROBIOLOGY, SOCIETY FOR GENERAL MICROBIOLOGY, GB, vol. 52, 1 January 2002 (2002-01-01), pages 639-646, XP002351633, ISSN: 1466-5026
- KROONEMAN, J. et al.: "Lactobacillus diolivorans sp. nov., a 1, 2-propanediol- degrading bacterium isolated from aerobically stable maize silage", International Journal of Systematic and Evolutionary Microbiology, vol. 52, 2002, pages 639-646, XP002351633, ISSN: 1466-5026
- MITSURU HONMA: "Niji Hakko o Yokusei suru 'Saimasuta SP' no Goshokai", Yuki Tane News, 1 July 2016 (2016-07-01), pages 6-7, XP009516104, Retrieved from the Internet: URL:https://www.snowseed.co.jp/wp/wp-conte nt/ uploads/seednews/368 03.pdf [retrieved on 2017-05-22]

## Description

### Technical Field

The present invention relates to a lactic acid bacterium for preparing silage, an additive for preparing feed or additive for preparing silage that uses such lactic acid bacterium, and a method for preparing silage using such additive.

### Background Art

Silage is an important feed and a main staple for cows, and the quality of silage has significant impact on dairy farming operations. Silage pertains to a technique of fermenting raw grass and other fodder crops for long-term preservation. However, two problems have long been discussed regarding the production of silage. One is the butyric acid fermentation caused by butyric acid bacteria during airtight preservation, which must be inhibited. The other is the secondary fermentation of silage caused by heating, and consequent deterioration of increasing quantities of yeast, etc., after opening, which must also be inhibited. To solve these two problems, silage additives have long been examined; however, no silage additive is available yet that can solve both problems. In particular, the issue of secondary fermentation is becoming more serious as the use of TMR feeds becomes wide-spread.

Proposals have been made to address these problems. One major proposal is to add a lactic acid bacterium, which is highly capable of producing lactic acid in silage, when the silage is prepared so that a drop in the pH value of the silage is promoted by the lactic acid, thereby inhibiting the growth of harmful microorganisms. Patent Literature I describes that a drop in the quality of silage due to harmful microorganisms can be prevented by using, as a silage lactic acid bacterium, the lactic acid bacterium Lactobacillus plantarum Chikuso-I strain (FERM P-18930) having excellent acid resistance and lactic acid fermentation ability or the lactic acid bacterium Lactococcus lactis RO50 strain (FERM P-18931) having antibacterial action against aerobic bacteria and butyric acid bacteria. However, there is no mention regarding the ability of the invention in Patent Literature 1 to inhibit secondary fermentation, and its effect in this regard is unknown.

Patent Literature 2 describes using, as a silage lactic acid bacterium, the lactic acid bacterium Enterococcus faecium NAS62 strain (NITE P-781) that produces bacteriocins, to inhibit harmful microorganisms through the antibacterial action of bacteriocins. Patent Literature 2 describes that filament bacteria are not detected in silage, and that filament bacteria are not detected in fermented TMR feeds, either.

Patent Literature 3 proposes an additive for preparing silage that contains a homofermentative lactic acid bacterium capable of producing Reuterin that has antimycotic action, as well as glycerol and vitamin B12. Reuterin is an antibacterial substance produced by Lactobacillus reuteri, in an anaerobic ambience, in a culture medium containing glycerin. β-hydroxypropionatdehyde, which is a fermentation product of glycerin, is detected in the supernatant of this culture, and this β-hydroxypropionatdehyde, which is presumably present in aqueous solutions in the form of a monomer, hydrate, or dimer, is called "Reuterin." Reuterin exhibits antibacterial property against Grampositive bacteria, Gram-negative bacteria, yeasts, and fungi. However, producing Reuterin is not very practical in actual silage preparation applications because it requires a large quantity of glycerol.

Patent Literature 4 discloses a composition for silage production that uses Lactococcus lactis capable of producing Nicin, which is an antibacterial substance, as well as a lactic acid bacterium resistant to lactic acids.

Patent Literature 5 describes that, when Lactobacillus diolivorans separated from silage was added to silage, the silage did not undergo abnormal fermentation.

As described above, various types of lactic acid bacteria for preparing silage, and of additives for manufacturing silage using these lactic acid bacteria, are proposed; however, the reality is that none of them is exactly satisfying. In particular, the issue of secondary fermentation of silage presents a big challenge.

Put simply, the issue of secondary fermentation of silage refers to an aerobic deterioration that occurs after the silage is taken out of a silo or wrapping roll. None of the silage lactic acid bacteria proposed by prior art has been able to completely solve this issue. In a sealed silo or wrapping roll, silage is in a constant, low-pH environment filled with carbonic acid gas, nitrogen gas, etc., and is stable. The various bacteria that had attached themselves to the material and entered the silo or roll together with the material when it was charged, are dormant in this condition. Once the silo is opened or roll is broken and the silage is exposed to air, however, the microorganisms that become active in the presence of air, or specifically aerobic microorganisms, grow quickly. In particular, yeasts quickly become active under suitable humidity, temperature, and nutrition (yeasts take the lactic acid, etc., produced in the silage as nutrients), and the silage temperature rises rapidly. Stimulated by this heat, fungi start growing and consequently the silage not only turns brown and gives off a foul smell, but it also becomes less appetizing to and digestible by cows, causes loss of dried matter of silage, causes diarrhea in milk cows, leads to production of alcohol insecurity milk, or the like, resulting in a significant monetary loss.

### Background Art Literature

### Patent Literature

Patent Literature 1: Japanese Patent Laid-open No. 2004-041064
Patent Literature 2: Japanese Patent Laid-open No. 201 1-41474
Patent Literature 3: Japanese Patent Laid-open No. 2008-35728
Patent Literature 4: International Patent Laid-open No. 2013/001862
Patent Literature 5: US Patent Laid-open No. 2005/0281917

### Summary of the Invention

The invention is defined in the appended claims.

### Problems to Be Solved by the Invention

One object of the present invention is to provide a lactic acid bacterium that inhibits secondary fermentation of silage and thus proves useful in ideal preparation of silage. Another object of the present invention is to provide a method for manufacturing silage whose secondary fermentation is inhibited.

### Means for Solving the Problems

After a great deal of research and repeated screenings of lactic acid bacteria for preparing silage and feeds to achieve the aforementioned objects, the inventors of the present invention identified a group of Lactobacillus diolivorans having anti-yeast action, from among the Lactobacillus diolivorans that were not heretofore drawing attention as lactic acid bacteria for preparing silage. The Lactobacillus diolivorans in this group were confirmed to be highly capable of producing lactic acid and acetic acid, to improve the quality of silage. The aforementioned objects were achieved by utilizing this group of Lactobacillus diolivorans.

The present invention encompasses the following constitutions.
(1) A lactic acid bacterium for preparing silage, constituted by a Lactobacillus diolivorans having anti-yeast action, wherein the Lactobacillus diolivorans is the Lactobacillus diolivorans SBS-0006 strain (NITE BP-02208), Lactobacillus diolivorans SBS-0007 strain (NITE BP-02209), or Lactobacillus diolivorans SBS-0009 strain (NITE BP-02210).
(2) Silage containing the lactic acid bacterium Lactobacillus diolivorans according to (1).
(3) An additive for preparing silage, which comprises the lactic acid bacterium Lactobacillus diolivorans according to (1).
(4) A method for preparing silage, characterized in that an additive for preparing silage according to (3) is added to the silage material.

### Effects of the Invention

The present invention can provide a Lactobacillus diolivorans, which is a lactic acid bacterium having anti-yeast action, as well as, in actual silage fermentation conditions, action to inhibit yeasts and other fungi and characteristics, to produce an antifungal substance in a culture solution of the lactic acid bacterium. This lactic acid bacterium can provide a silage additive that offers excellent acid resistance and can achieve quality lactic acid fermentation. Secondary fermentation is inhabited in the obtained silage. This means that, when the present invention is applied to TMR feeds, etc., the feeds are expected to give off less decomposition smell or deterioration smell, be very safe for and eaten more by livestock, and increase the weight of fattening cattle as well as the amount of milk produced by milk cows, among others.

### Brief Description of the Drawings

[FIG. 1] A graph showing that crushed bacteria cells of the lactic acid bacterium Lactobacillus diolivorans proposed by the present invention inhibits growth of the yeast Pichia fermentans.
[FIG. 2] A graph showing that crushed bacteria cells of the lactic acid bacterium Lactobacillus diolivorans proposed by the present invention inhibits growth of the yeast Issatchenkia orientalis.
[FIG. 3] Results of a lactic acid bacterium Lactobacillus diolivorans screening test, where inhibition of growth of the yeast Pichia fermentans is an indicator.
[FIG. 4] Results of a lactic acid bacterium Lactobacillus diolivorans screening test, where inhibition of growth of the yeast Issatchenkia orientalis is an indicator.
[FIG. 5] A scatter graph showing the results of screening tests shown in FIGS. 3 and 4. This graph is used to evaluate the anti-yeast effects against the two yeasts at a glance.
[FIG. 6] A graph showing the fermentation qualities of the silage samples fermented at 25°C after adding the lactic acid bacteria for silage in Examples 1 to 4 and Comparative Example.
[FIG. 7] A graph showing the fermentation qualities of the silage samples fermented at 15°C after adding the lactic acid bacteria for silage in Examples 1 to 4 and Comparative Example.
[FIG. 8] A graph showing the times taken by the silage samples prepared by adding the lactic acid bacteria for silage in Examples 1 to 4 and Comparative Example, to reach 30°C after opening.
[FIG. 9] A graph showing the times taken by the silage samples prepared by adding the lactic acid bacteria for silage in Examples 1 to 3 and Comparative Examples 1 and 2 and then stored under a condition of 15°C, to reach 30°C after opening.

### Mode for Carrying Out the Invention

The invention is defined in the appended claims,

The present invention relates to a new lactic acid bacterium, Lactobacillus diolivorans, having anti-yeast action.

Also, the present invention relates to an additive for preparing feed that contains the aforementioned lactic acid bacterium, a feed such as silage characterized by containing a strain of the bacterium, and a method for preparing the same.

The present invention is explained in detail below.

The lactic acid bacterium Lactobacillus diolivorans proposed by the present invention is a lactic acid bacterium that undergoes hetero-fermentation. It can be obtained by selecting this lactic acid bacterium as it separates from silage.

Normally, from among the lactic acid bacterial strains separated from silage, strains classified as Lactobacillus diolivorans are mixed with shreds of dent corn or other plant material used for preparing silage, as well as a culture solution of a yeast separated from silage, and the mixture is placed in polyethylene pouches and then stored for two months at 25°C, after which the pouches are opened and the organic acid content, yeast cell count, lactic acid bacteria cell count, and time to reach 30°C after opening (secondary fermentation time) are used as indicators to conduct a primary screening.

Next, those Lactobacillus diolivorans exhibiting favorable organic acid content, yeast cell count, lactic acid bacteria cell count, and time to reach 30°C after opening (secondary fermentation time) for silage, are cultured using the MRS culture medium or GYP culture medium, after which the bacteria cells are collected and then broken into fragments, and these fragmented bacteria are selected after confirming their anti-yeast action (action to inhibit yeast growth) using growth of the Pichia membranifaciens, Issatchenkia orientalis, or Pichia fermentans yeast separated from silage, as an indicator.

Among the anti-yeast Lactobacillus diolivorans having the aforementioned characteristics, those exhibiting high lactic acid production, as well as silage fermentation action of favorable quality characteristics, in addition to anti-yeast action, are preferred.

The invention provides the Lactobacillus diolivorans SBS-0006 strain (NITE BP-02208), Lactobacillus diolivorans SBS-0007 strain (NITE BP-02209), and Lactobacillus diolivorans SBS-0009 strain (NITE BP-02210), which were separated by the inventors of the present invention and internationally deposited with the National Institute of Technology and Evaluation's Patent Microorganisms Depositary (NPMD) (Suite 122, 2-5-8 Kazusakamatari, Kisarazu-shi, Chiba-ken, Japan (Postal Code 292-0818)) effective February 22, 2016.

The above lactic acid bacteria can be cultured using a standard culture medium for lactic acid bacteria. The culture medium is not limited in any way, and any culture medium used for culturing lactic acid bacteria may be used. For example, the GYP culture medium or MRS culture medium may be used. While the culture conditions are not limited in any way, normally the culturing is performed at pH 5.0 to 7.0 and 25 to 40°C for 10 to 48 hours. The cultured lactic acid bacteria, which may be in a state of culture solution or concentrated solution in which the bacteria cells are concentrated, may be added to the material for preparing silage, or to fermented silage, TMR, or other fermented feeds. These solutions may be used frozen. Also, the cultured lactic acid bacteria may be freeze-dried, spray-dried or fluid-bed-dried, together with an appropriate protective agent and a base material, into a powder state and used as an additive for preparing silage. If necessary, trehalose, calcium carbonate powder, etc., may be added to the freeze-dried lactic acid bacteria powder. Also, any known component, such as cellulase, may be added to promote the fermentation of silage.

The additive for preparing silage proposed by the present invention may be used to prepare various types of silage and fermented feeds. The material used for preparing silage or fermented feed is not limited in any way, and any material normally used as a feed may be used, where examples include pasture grasses and fodder crops such as alfalfa, clover, Timothy grass, orchard grass, reed canary grass, quitch grass, Italian rye grass, perennial rye grass, tall fescue, meadow fescue, festulolium, Kentucky blue grass, red top, Guinea grass, rose grass, Napier grass, oats, barley, rye, sorghum, Sudan grass, millet, corn, fodder rice, as well as soft grains primarily comprising grains of corn and rice. Among food production byproducts, examples include beer lees, low-molt beer lees, tofu lees, tea lees, shochu lees, whiskey lees, beet pulp, bagasse, coffee lees, juice lees, kale lees, starch lees, etc. Among agricultural byproducts, examples include rice straw, straw, out-of-spec vegetables, etc. Also, the present invention may be added to fermented TMR prepared by mixing food production byproducts, silage, agricultural biproducts, hay, concentrated feeds, vitamin/mineral formulations, etc. Ideally these silage and fermented feed materials are used by adjusting the moisture content to a range of 40 to 90 percent by mass.

As for the use quantity of an additive for preparing silage, the number of bacteria cells to be added is adjusted so that the total cell count will become 10³ to 10⁷, or preferably 10⁴ to 10⁶, per 1 gram of material. Under the present invention, the additive is not limited in any way and any pasture grass or fodder crop used for silage or fermented feed may be used, where examples include alfalfa, clover, Timothy grass, orchard grass, reed canary grass, quitch grass, Italian rye grass, perennial rye grass, tall fescue, meadow fescue, festulolium, Kentucky blue grass, red top, Guinea grass, rose grass, Napier grass, and other pasture grasses, as well as corn, sorghum, and other fodder crops. Ideally, corn or sorghum that easily undergoes secondary fermentation is used. As for the form of pasture grasses and fodder crops, freshly cut grass may be used directly, or refrigerated or frozen and then preserved before use, or air-dried or freeze-dried pasture grass may be crushed and used as dry powder. Also, commercially available feeds such as Lucerne pellets and hay cubes may be used, or soft grains primarily comprising grains of corn and rice, may be used.

Silage and fermented feeds are fermented under anaerobic conditions. The fermentation container is not limited in any way and any container capable of maintaining an anaerobic state to some extent may be used, where examples include a bunker silo, stack silo, trench silo, tower silo, underground silo, block silo, cup silo, roll bale, flexible container bag, etc. Silage can be obtained by letting the material stand for one week to two months at an outside temperature of normally 5 to 30°C, or preferably 15 to 25°C, for fermentation.

Silage and fermented feeds obtained by using the additive for preparing silage proposed by the present invention do not undergo secondary fermentation after opening, and thus are preferred and eaten in favorable volumes by cows, and their nutritional values do not drop.

### Examples

The present invention is explained in greater detail below by citing examples and comparative examples. It goes without saying that the following descriptions are provided to explain the present invention and they do not limit the present invention to these examples.

### <1. Primary Screening of Lactobacillus Diolivorans>

Eighteen lactic acid bacterial strains that had been separated from silage and confirmed to be Lactobacillus diolivorans by Snow Brand Seed Co., LTD., were used. The names of the strains given by Snow Brand Seed Co., LTD. are as follows: 3,66, 126, 128, 183, 187, 379, 518, 547, 574, 586, 602, 603,617, 707, 746, 767, and Oda 2.

### (1) Preparation of Silage

The 18 strains were cultured in the GYP liquid culture medium and 1.5 × 10⁹ CFU/mL of culture liquid was prepared and freeze-dried, to obtain lactic acid bacteria powders. A primary screening test was conducted on these powders.

In the primary screening test, 3 mL of a yeast culture liquid (7.0 × 10⁸ CFU/mL) separated from existing silage was added to 300 g of shreds of dent corn harvested in the fall and then frozen and preserved as silage material, after which 1 mL of a solution prepared by suspending 30 mg of each of the lactic acid bacteria powders in 100 mL of ion exchanged water was added by spraying (by 1 mg/kg equivalent), and the ingredients were mixed, weighed, and separated into units of 100 g, which were then individually sealed in polyethylene pouches (repeated three times). The sealed pouches were preserved for two months under a silage preparation condition of 25°C.

### (2) Quality of Silage

After the prescribed period, the pouches were opened to remove the silage for measurement of pH, organic acid contents (by HPLC), lactic acid bacteria cell count, and yeast cell count. In addition, the rise in silage temperature after opening was measured at a room temperature of 25°C, to measure how long it took to reach 30°C, an indicator for secondary fermentation of silage. The measured results are shown in Table 1 below.

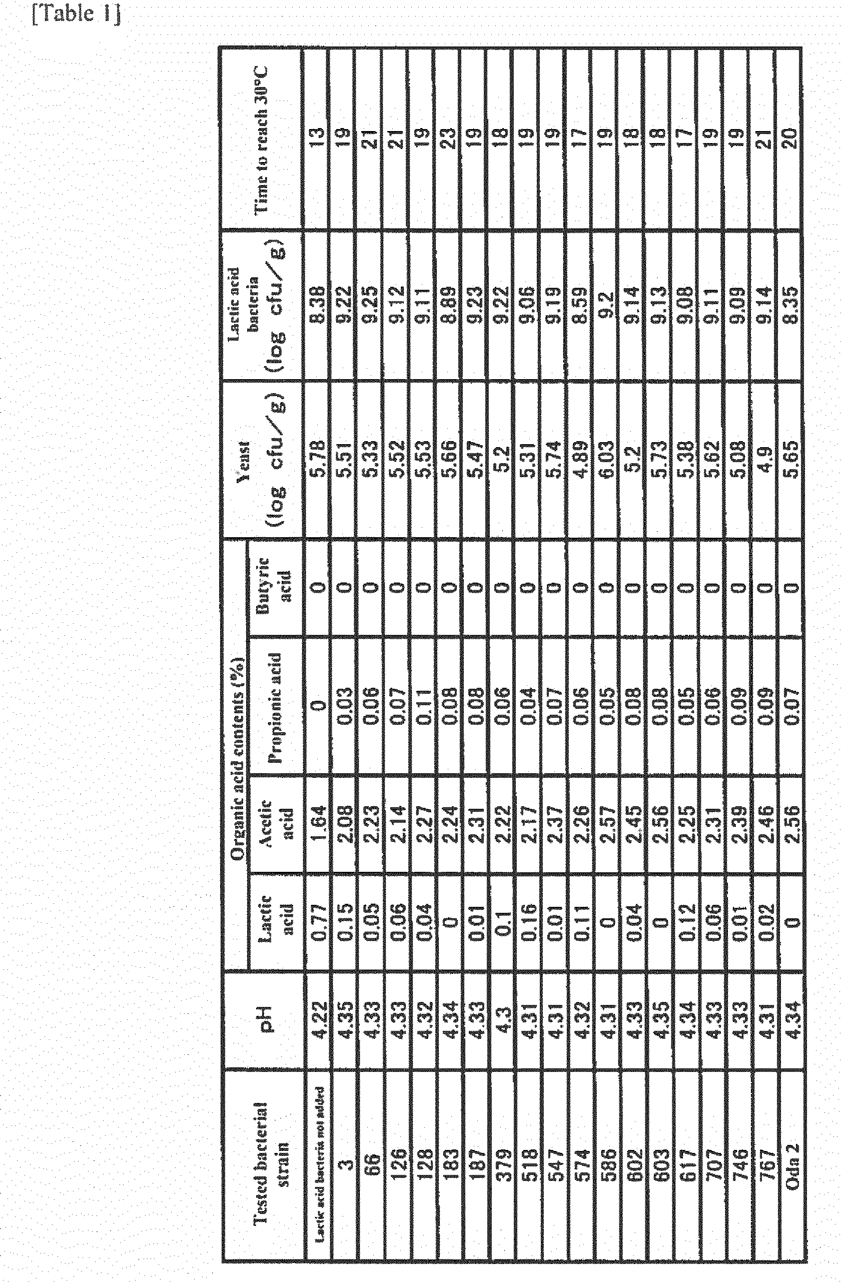

As shown in Table 1, the 18 tested strains of Lactobacillus diolivorans were confirmed to be favorable strains that produced acetic acid/propionic acid, but did not produce butyric acid, in the silage. However, these silage samples made from frozen and preserved dent corn primarily contained acetic acid, and their secondary fermentation could not be prevented by means of yeast inhibition.

### <2. Secondary Screening of Lactobacillus Diolivorans>

Dent corn silage was prepared using the 18 strains of Lactobacillus diolivorans used in the primary screening, and a secondary screening was performed based on the time to reach 30°C due to secondary fermentation after opening, as an indicator.

### (1) Preparation of Silage

Dent corn (in the yellow ripe stage) was harvested and made into small pieces using a crusher, after which each of the lactic acid bacteria powders was added by 1 mg per 1 kg of dent corn and mixed well, just like in the primary screening, and 800 g of the mixture was filled and sealed in 1-L bottles as silage. The silage was stored at a temperature of 25°C and fermented for 10 weeks.

### (2) Screening of Bacterial Strains Based on Change in Silage Quality

After the prescribed period, the bottles were opened to remove the silage for measurement of change in silage temperature after opening, in order to measure how long it took to reach 30°C, an indicator for secondary fermentation of silage, to select bacterial strains that inhibited secondary fermentation. As a result, it was confirmed that the two strains of 603 and 767, in particular, prevented the silage temperature from rising for a long time (100 hours or longer) after opening. Also, yeast growth was confirmed to be inhibited in the silage prepared with the 603 and 767 strains.

### <3. Separation of 767S Strain and 767R Strain from 767 Strain>

When the 767 strain was sub-cultured in the MRS plate medium, it separated into a clone having a smooth colony shape and a clone having a rough colony periphery. The former was named "767S," and the latter was named "767R."

The tests explained below were conducted on the three strains of 767S, 767R and 603.

### <4. Yeast Growth Inhibition Test>

The 767S, 767R, and 603 strains inhibit yeast growth in silage, and in pure yeast culture systems, crushed bacteria of the 767S, 767R, and 603 strains inhibit proliferation. This is explained by citing representative examples.

### (1) Test Method

### 1) Preparation of Crushed Lactic Acid Bacteria Cell Solution

An MRS (Difco) liquid culture medium and a corn powder 10% broth culture medium were mixed at a ratio of 1:1. The lactic acid bacteria were inoculated into the mixed medium and cultured for five days at 37°C. Thereafter, 10 mL of the culture solution was dispensed into a 15-mL Falcon tube and centrifuged at 3500 rpm for 15 minutes. Next, the supernatant was discarded and the sediments were washed twice with PBS (-). The washed sediments were centrifuged again at 3500 rpm for 15 minutes, and the sediments were suspended in 1 mL of PBS (-). A 50-mL Falcon tube containing water was put in a tray filled with ice, and the 15-mL Falcon tube was placed therein, and in this condition, the cells were crushed for 5 minutes using an ultrasonic disintegrator. After a centrifugation at 3500 rpm for 5 minutes, the supernatant was filtered through a 0.2-µm membrane filter and sterilized, to obtain a crushed bacteria cell solution. This lactic acid bacteria extraction liquid was used for testing. It should be noted that the lactic acid bacteria on which this test was conducted, were those known to inhibit yeast growth in silage, or namely Lactobacillus paracasei 2347, Lactobacillus buchneri NK01, and Lactococcus lactis TJ48 (SBS0001), as well as the 767S and 767R strains separated above.

### 2) Yeast Growth Inhibition Test

Onto a 96-well microplate, 180 µL of yeast nitrogen base (Difco) adjusted to pH 4.0, 10 µL of 100-mM carbon source, and 10 µL of each solution of crushed test lactic acid bacteria cells, were dispensed, and then 2 µL of each yeast washed with PBS (-) (culture solution obtained by culturing the yeast at 27°C for 48 hours in the YPD culture medium (yeasts: Pichia fermentans (hereinafter referred to as "Pf") and Issatchenkia orientalis (Candida krusei, hereinafter referred to as "Ck")) was added. The carbon source was lactic acid for Pf, and acetic acid for Ck. It should be noted that both of the yeasts used had been separated from silage, and identified, by the inventors of the present invention.

The culture solution was aerobically cultured at 27°C for 48 hours and then agitated by pipetting, after which the yeast growth inhibition action was evaluated based on O.D. 600 nm measurement.

### (2) Results

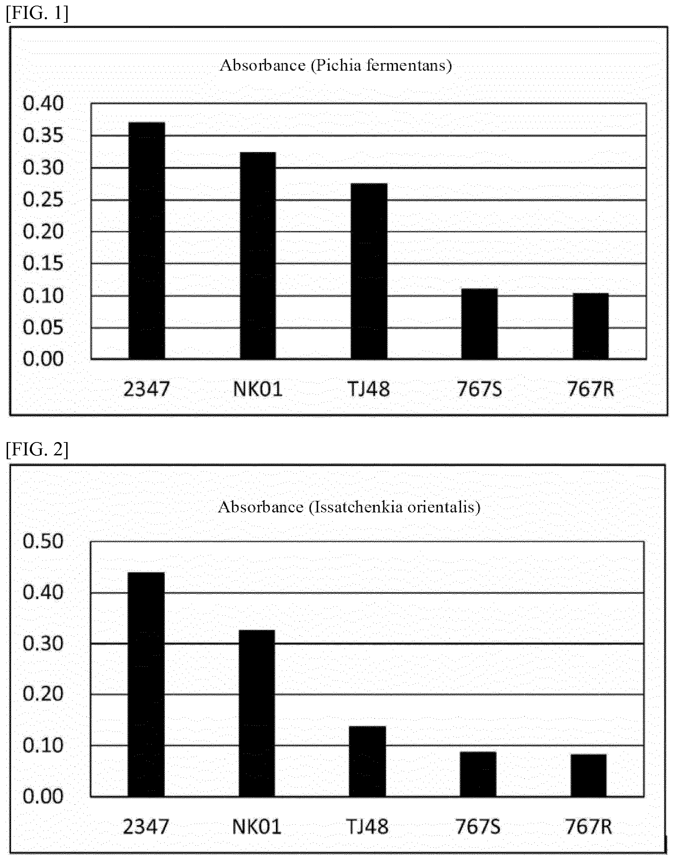

The growth inhibition test results with respect to yeast Pf are shown in FIG. 1, while the growth inhibition test results with respect to yeast Ck are shown in FIG. 2.

Clearly the crushed bacteria cell solutions of 767S and 767R strains were confirmed to inhibit yeast growth compared to those of other lactic acid bacteria. This test was determined useful for screening of anti-yeast action.

Also, when the same test was conducted on the 603 and 547 strains screened based on their ability to inhibit the rise in silage temperature, the crushed bacteria cell solutions of 603 and 547 strains were also confirmed to inhibit yeast growth markedly. Presumably this yeast inhibition action is attributable to production of a substance that exhibits some kind of anti-yeast action in the lactic acid bacteria cells.

No Lactobacillus diolivorans have been known that produce such substance having anti-yeast action.

The above test results confirm that the Lactobacillus diolivorans separated from silage and screened by the inventors of the present invention represent a new group of microorganisms having good silage fermentation capability as well as anti-yeast action.

It should be noted that the 767S, 767R, and 603 strains have been internationally deposited by the applicants of the present invention with the National Institute of Technology and Evaluation's Patent Microorganisms Depositary as the Lactobacillus diolivorans SBS-0006 strain (NITE BP-02208), Lactobacillus diolivorans SBS-0007 strain (NITE BP-02209), and Lactobacillus diolivorans SBS-0009 strain (NITE BP-02210), respectively.

### <5. Bacterial Properties of 603, 547, 767S, and 767R Strains>

The four strains of 603, 547, 767S, and 767R above are classified as Lactobacillus diolivorans. These four strains were further examined for their bacterial properties (sugar assimilation properties) according to Bergey's Manual. Their assimilation properties after 48 hours of culturing were different from those exhibited after 5 days of culturing. Table 2 below shows the assimilation properties after 48 hours of culturing, while Table 3 shows the assimilation properties after 5 days of culturing.

It should be noted that Table 2 also lists the sugar assimilation properties of the JCM12183 strain (provided by RIKEN BioResource Center), which is a standard Lactobacillus diolivorans strain whose information is available to the public, for comparison.

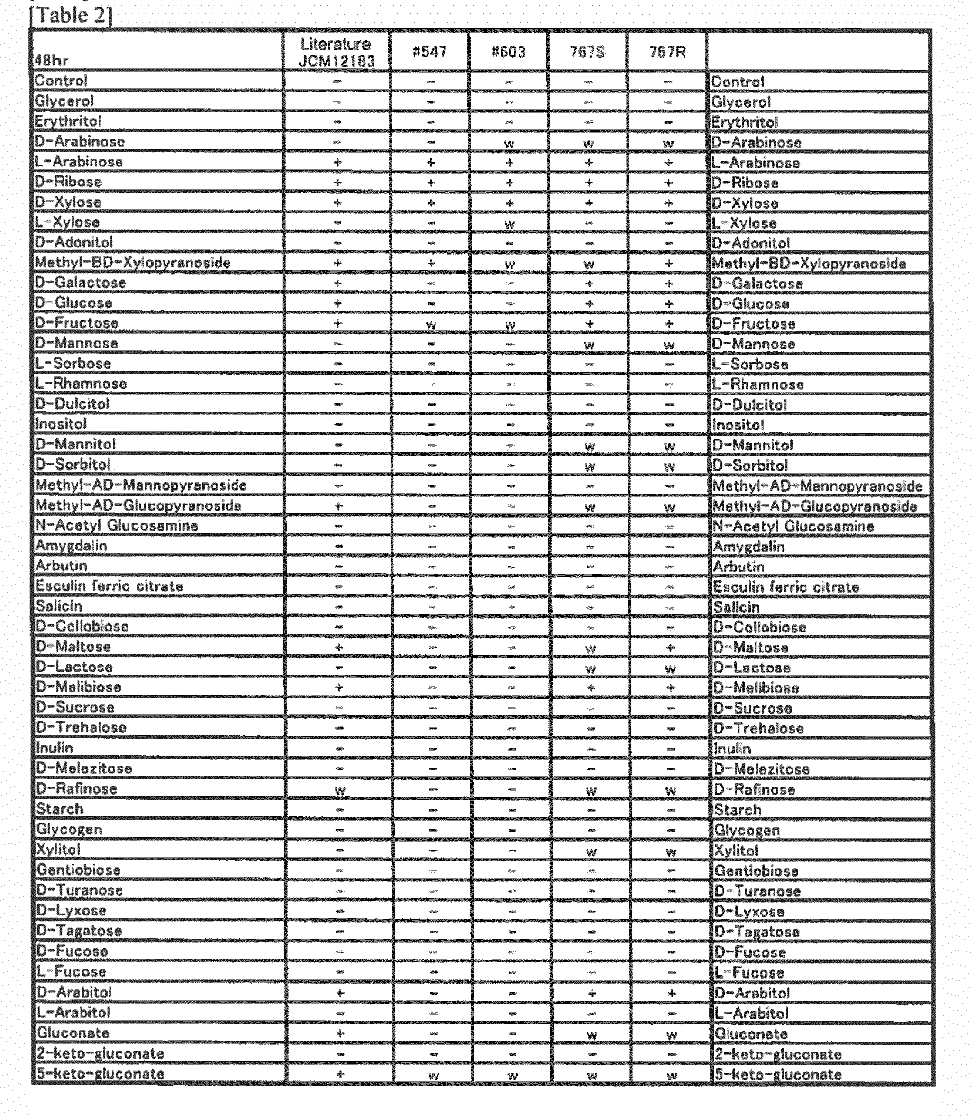

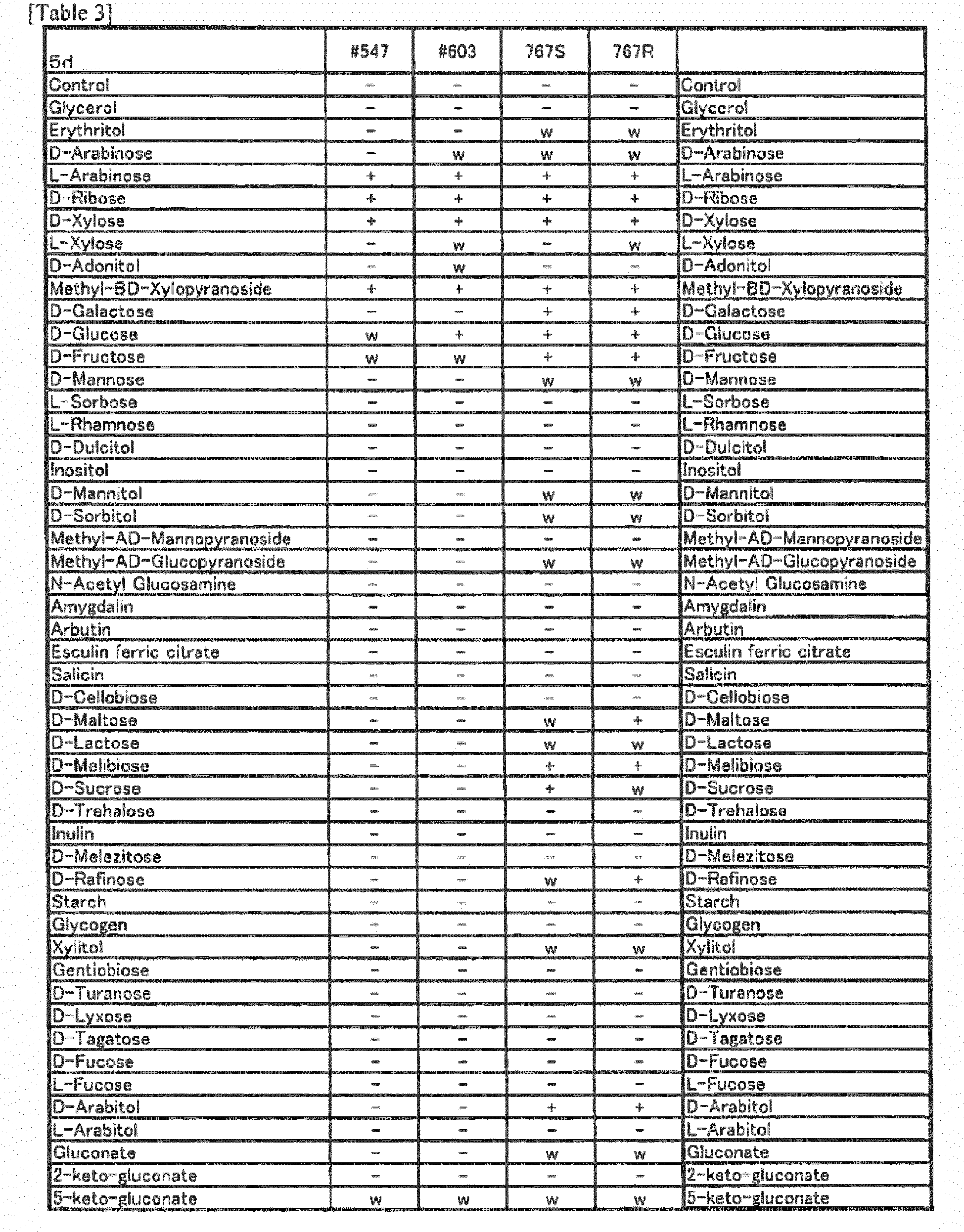

It should be noted that the 767S and 767R strains are confirmed to have lactose assimilation property, as well as sucrose assimilation property not shown by the standard strain. In addition, the 547 and 603 strains are characterized by low galactose and glucose assimilation properties.

### <6. Tertiary Screening of Lactobacillus Diolivorans>

As described above, the 767S and 767R strains were selected, through the secondary screening, based on their ability to inhibit yeast in silage. This test was again applied to preserved strains of Lactobacillus diolivorans that had been separated from silage by the inventors of the present invention, for a tertiary screening based on anti-yeast action. The strains subjected to the test are as follows:
The 16 strains of 126, 128, 183, 187, 518, 547, 574, 586, 602, 603, 617, 707, 746, 767S, 767R, and Oda 2 were tested.

### (1) Test Method

### 1) Preparation of Crushed Lactic Acid Bacteria Cell Solution

As in the test in 4 above, 100 µL of an MRS culture solution of each Lactobacillus diolivorans was inoculated into a 1:1 mixture of MRS (Difco) liquid culture medium and corn powder 10% broth culture medium, and cultured at 37°C for five days.

The culture solution was centrifuged at 3000 G for 15 minutes, after which the supernatant was discarded, I mL of PBS (-) was added to the sediments, and the cells were crushed using an ultrasonic disintegrator under ice cooling. The crushed cell solution was centrifuged at 3000 G for 15 minutes, and the supernatant of the crushed cell solution was filtered through a 0.22-µm membrane filter and sterilized.

Next, 10 µL of the aforementioned supernatant, 180 µL of YNB culture medium (pH 4.0) containing 0.1% glucose, and 100 µL of 100 mM carbon source (lactic acid or acetic acid), were added onto a 96-well microplate, into which 2 µL of YPD culture solution of yeast was inoculated and cultured at 27°C for 48 hours. For the yeast, Pichia fermentans (Pf) was used when lactic acid was used as the carbon source, while Issatchenkia orientalis (Ck) was used when acetic acid was used as the carbon source.

At the end of culturing, each well was fully mixed/agitated by pipetting and absorbance was measured at a wavelength of 600 nm using a microplate reader. Based on the earlier anti-yeast test, a screening result of 0.35 or lower absorbance in both the lactic acid culture medium and the acetic acid culture medium was determined as an indication of anti-yeast/yeast inhibition action.

### 2) Results

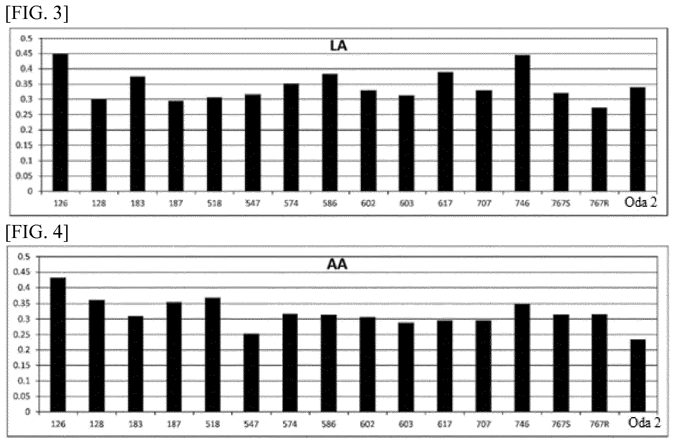

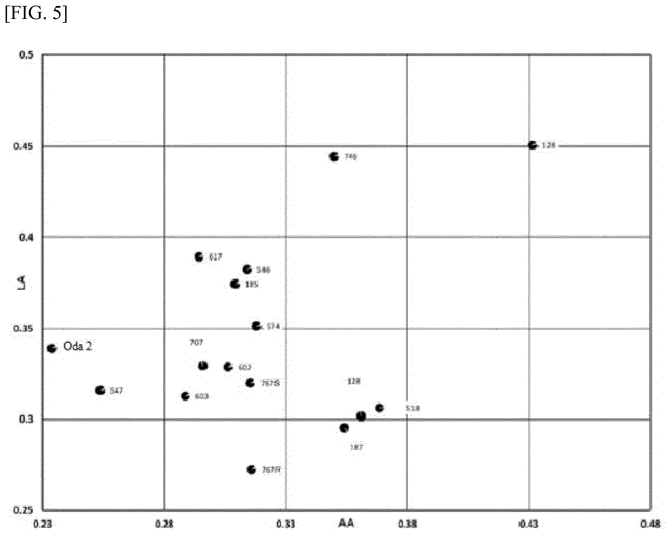

FIG. 3 shows the evaluation (absorbance) of anti-yeast action based on lactic acid (LA) as the carbon source, while FIG. 4 shows the evaluation (absorbance) of anti-yeast action based on acetic acid (AA) as the carbon source. Also, a scatter graph was created, with the X-axis representing the AA absorbance and the Y-axis representing the LA absorbance. This scatter graph is shown in FIG. 5. By using the scatter graph, effects against yeast species of different types can be determined simultaneously.

As is evident from FIGS. 3, 4, and 5, the 547, 602, 603, 707, 767S, and 767R strains were screened as Lactobacillus diolivorans strains meeting the yeast growth indicator of 0.35 or lower absorbance.

In addition, the test results using the examples shown below revealed that Lactobacillus diolivorans with anti-yeast action can be screened using a test yeast of Pf or Ck. In other words, the aforementioned anti-yeast effectiveness test is an excellent method to screen for lactic acid bacteria having an action to inhibit secondary fermentation of silage (rise in silage temperature) after opening.

### <6. Manufacturing of Silage Additives Using 767S, 767R, 603, and 547 Strains>

### 1. Culturing of Bacteria Cells

In the examples below, silage additives were manufactured using the 767S, 767R, 603, and 547 strains by considering the rate of growth of lactic acid bacteria, etc.

The glucose yeast extract peptone liquid culture medium shown in Table 4 below was adjusted to pH 5.5, and then sterilized at 121°C for 15 minutes. The culture medium was prepared in different quantities of 10 mL for activation culturing, 8 L for inoculation, and 1600 L for main culturing.

One inoculation loop of cells of each bacterium preserved at -80°C was inoculated into the activation culture medium and cultured at 37°C for 24 hours. At the end of culturing, 8 mL of the activation culture solution was inoculated into 8 L of the inoculation medium and cultured at 37°C for 24 hours. Again, at the end of culturing, the entire quantity of the inoculation culture solution was added to 1600 L of the main culture medium and cultured at 37°C for 24 hours.

**[Table 4]**

| Component | Ratio |
|---|---|
| Glucose | 2.00% |
| Yeast extract | 1.00% |
| Peptone | 1.00% |
| Iron sulfate | 0.01% |
| Magnesium sulfate | 0.01% |
| Manganese sulfate | 0.01% |

Culture medium composition

### 2. Freeze-drying of Bacteria Cells

As a result of main culturing, a culture solution of 6.0 × 10⁹ CFU/mL in bacteria cell density was obtained by 1600 L. All of the culture solution was centrifuged in a continuous centrifuge, to obtain a bacteria cell concentrate of 1.0 × 10¹¹ CFU/mL by 40 L. The 40 L of bacteria cell concentrate was added to 80 L of a 15% trehalose solution that had been sterilized at 121°C for 15 minutes and then let cool, after which the mixture was suspended at 15°C for 30 minutes, frozen at -30°C, and preserved in a frozen state until it was eventually freeze-dried. The frozen bacteria cells were freeze-dried using ordinary methods. The obtained freeze-dried powder had a live cell count of 2.4 × 10¹¹ CFU/g.

### 3. Manufacturing of Powder for Preparing Silage

The collected freeze-dried powder was measured for live cell count, and then mixed with a trehalose powder until 2 × 10¹¹ CFU/g was achieved. The powders thus prepared from the 767S strain, 767R strain, 603 strain, and 547 strain are referred to as Example 1, Example 2, Example 3, and Example 4, respectively, below.

It should be noted that these powders also represent core materials of powders for preparing silage. They are diluted by 5- to 1000-fold using a trehalose, calcium carbonate, zeolite, or other powder, to obtain silage additives.

### <7. Effectiveness Test of Powder for Preparing Silage (I)>

### (1) Test Samples

The powders in Examples 1 to 4 were used to prepare silage for the purpose of effectiveness confirmation. In addition, a powder prepared in the same way from the Lactobacillus buchneri NK-01 strain used in "Silo SP," a lactic acid bacteria formulation for preparing silage sold by Snow Brand Seed Co., LTD., was used as a comparative example.

### (2) Preparation of Silage

In a process of preparing corn silage, each of the lactic acid bacteria powders in Examples 1 to 4 and Comparative Example was added and mixed until a lactic acid bacteria cell count of 10⁶ CFU/g per silage material was achieved, after which the mixture was filled and sealed in 1-L test bottles by approx. 800 g each and preserved for two months under two conditions of 25°C and 15°C, to prepare silage. After the two months, the bottles were opened and the following tests were conducted. It should be noted that three test bottles were prepared in each example.

### (3) Silage Quality Test

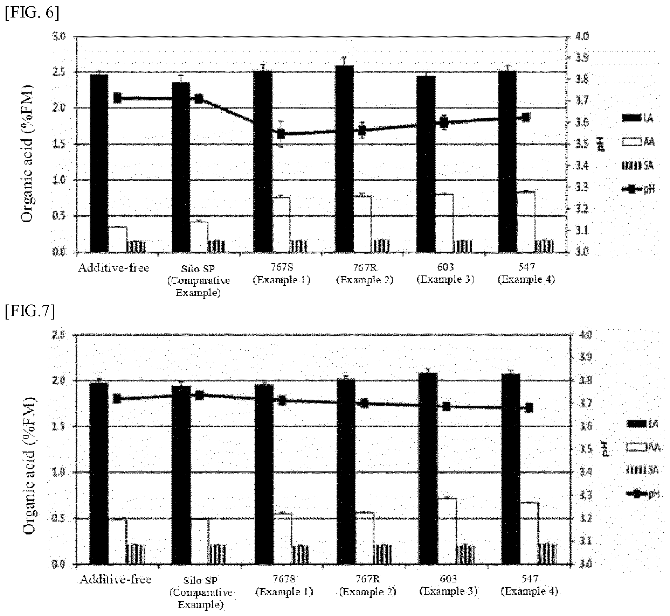

Abnormal fermentation was not observed in any of the bottles. Each silage was measured for pH, lactic acid content (LA), acetic acid content (AA), and succinic acid content (SA) using ordinary methods, to evaluate the quality of the silage. The level of fermentation smell was also checked. FIG. 6 shows the silage qualities after preservation at 25°C, while FIG. 7 shows the silage qualities after preservation at 15°C.

All of the 25°C-preserved silage samples in Examples 1 to 4 showed a lower pH, and were thus better, than the sample in Comparative Example 1 and the additive-free sample. It was confirmed that they also had a good silage smell and did not exhibit abnormal fermentation.

The 15°C-preserved silage samples in the Examples and Comparative Example did not show notable difference in quality.

### (4) Secondary Fermentation Test after Opening

Time to reach 30°C after opening, yeast cell count immediately after opening, yeast cell count of 25°C-preserved silage 2 days after opening, yeast cell count of 15°C-preserved silage 3 days after opening

### 1) Time to Reach 30°C

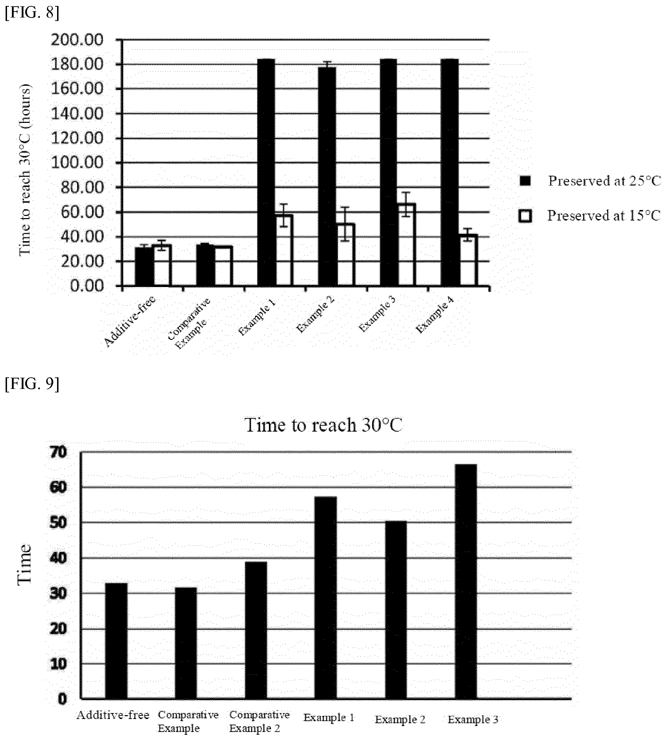

The silage samples in the Examples and Comparative Example, and the additive-free sample, were measured for time to reach 30°C. The measured results are shown as averages and standard deviations. Also, the statistical significance test was conducted on each example based on Student's t-test (significance level: 5%). The results are shown in Table 5 below and FIG. 8.

**[Table 5] Time to reach 30°C (hours until secondary fermentation)**

| | Preserved at 25°C | | | Preserved at 15°C | | |
|---|---|---|---|---|---|---|
| | Hours | Slandard deviation | t-test | Hours | Standard deviation | t-test |
| Additive-free | 31.33 | 2.89 | a | 33 | 4 | a |
| Comparative Example (Silo SP) | 33.67 | 1.15 | a | 31.67 | 0.47 | a |
| Example 1 (767S) | 184 | 0 | b | 57.33 | 8.99 | ab |
| Example 2 (767R) | 177.67 | 5.69 | b | 50.33 | 15.57 | ab |
| Example 3 (603) | 184 | 0 | b | 66.33 | 9.84 | b |
| Example 4 (547) | 184 | 0 | b | 41.67 | 4.99 | ab |

| | | | | | | |
|---|---|---|---|---|---|---|
| In t-test, significant differences are present among the groups of different symbols. | | | | | | |

As shown in Table 5 and FIG. 8, secondary fermentation was markedly inhibited in the silage samples to which the lactic acid bacteria additive formulations for silage, provided in the Examples, were added. In addition, this effect was prominent in the silage samples preserved at 25°C, which is a preferred silage preparation temperature, with the silage in the Examples requiring around six times longer than the silage in the Comparative Example, to reach 30°C. This substantiates that the samples conforming to the present invention, as provided in the Examples, have an action to inhibit secondary fermentation of silage.

Based on the silage preserved at 15°C, the samples in the Examples took around 20 hours longer than the samples in the Comparative Example, and the additive-free control sample, before experiencing secondary fermentation.

### 2) Yeast Cell Counts of 25°C-preserved Silage Immediately after Opening and 2 Days after Opening

The silage samples in the Examples and Comparative Example, and the additive-free sample, were preserved at 25°C, and then measured for yeast cell count immediately after opening and also 2 days after opening. The measured results are shown as averages and standard deviations. Also, the statistical significance test was conducted on each example based on Student's t-test (significance level: 5%). The results are shown in Table 6 below.

**[Table 6] Yeast cell count, preserved at 25°C**

| | Immediately after opening | | | 2 days after opening | | |
|---|---|---|---|---|---|---|
| | Yeast cell count Log (cfu/g) | Standard deviation | t-test | Yeast cell count Log (cfu/g) | Standard deviation | t-test |
| Additive-free | 4.85 | 0.08 | c | 8.54 | 0.03 | b |
| Comparative Example (Silo SP) | 3.6 | 1.73 | bc | 8.25 | 0.27 | b |
| Example 1 (767S) | 1.6 | 0 | a | 2.94 | 0.58 | a |
| Example 2 (767R) | 1.6 | 0 | a | 2.94 | 0.58 | a |
| Example 3 (603) | 1.6 | 0 | a | 2.6 | 0 | a |
| Example 4 (547) | 1.6 | 0 | a | 2.6 | 0 | a |

| | | | | | | |
|---|---|---|---|---|---|---|
| In t-test, significant differences are present among the groups of different symbols. | | | | | | |

Table 6 shows that, with the silage samples to which the lactic acid bacteria additive formulations for silage, provided in the Examples, were added, the yeast cell count immediately after opening was as low as 1/1,000 or less of the levels of the silage sample in the Comparative Example and of the control sample (additive-free). As a result, the yeast cell count 2 days after opening was as low as 1/100,000 or less of the levels of the silage sample in the Comparative Example and of the control sample.

### 3) Yeast Cell Counts of 15°C-preserved Silage Immediately after Opening and 3 Days after Opening

The silage samples in the Examples and Comparative Example, and the additive-free sample, were preserved at 15°C, and then measured for yeast cell count immediately after opening and also 3 days after opening. The measured results are shown as averages and standard deviations. Also, the statistical significance test was conducted on each example based on Student's t-test (significance level: 5%). The results are shown in Table 7 below.

**[Table 7] Yeast call count, preserved at 15°C**

| | Immediately after opening | | | 3 days after opening | | |
|---|---|---|---|---|---|---|
| | Yeast cell count Log (cfu/g) | standard deviation | t-test | Yeast cell count Log (cfu/g) | Standard deviation | t-test |
| Additive-free | 5.36 | 0.22 | ab | 8.44 | 0.23 | ab |
| Comparative Example (Silo SP) | 5.48 | 0.16 | b | 8.49 | 0.2 | b |
| Example 1 (767S) | 3.44 | 0.73 | ab | 7.11 | 1.11 | ab |
| Example 2 (767R) | 4.79 | 1.45 | ab | 7.82 | 0.51 | ab |
| Example 3 (603) | 3.04 | 1.28 | a | 5.82 | 1.93 | a |
| Example 4 (547) | 4.99 | 0.29 | ab | 8.1 | 0.32 | ab |

| | | | | | | |
|---|---|---|---|---|---|---|
| In t-test, significant differences are present among the groups of different symbols. | | | | | | |

As shown in Table 7, the yeast cell count was lower with all of the silage samples to which the lactic acid bacteria additive formulations for silage, provided in the Examples, were added, compared to the silage sample in the Comparative Example; however, the effect was not very noticeable. The same is true with the yeast count 3 days after opening.

### <8. Effectiveness Test of Powder for Preparing Silage (II) (Test of Silage Fermented at 15°C)>

Another test was conducted regarding the effectiveness of the samples conforming to the present invention, to inhibit secondary fermentation that would otherwise occur in silage preparation at low temperature.

### (1) Test Samples

The powders in Examples 1 to 3 and in Comparative Example 1 were used to prepare silage for the purpose of effectiveness confirmation.

In addition, silage was prepared using a powder prepared in the same way from the JCM 12183 standard Lactobacillus diolivorans strain (provided by RIKEN BioResource Center) used in the present invention, as Comparative Example 2.

### (2) Test Method

Silage was prepared in the same manner as in the test in 7, wherein the silage was preserved for two months at 15°C, which is a silage fermentation condition in cold regions. The silage was opened after two months and subjected to the secondary fermentation test after opening. In other words, the time to reach 30°C after opening was measured in the same manner as in the test in 7.

### (3) Test Results

The times to reach 30°C, based on averages of three bottles, are shown in FIG. 9, The additive-free sample and sample in Comparative Example 1 reached 30°C in approx. 30 hours. The sample in Comparative Example 2 reached 30°C in 39 hours. On the other hand, the samples in Examples 1 to 3 took 50 hours or more to reach 30°C, according to the results. In other words, the silage additives according to the present invention inhibited secondary fermentation of the silage prepared at cold temperature, more effectively than the known silage preparation bacterial strain Lactobacillus buchneri NK-01, or the standard Lactobacillus diolivorans strain JCM12183.

The lactic acid bacteria additive formulations for silage according to the present invention inhibited yeast growth in silage, rise in silage temperature due to secondary fermentation, and drop in silage quality after opening. In particular, they were confirmed to achieve these results very effectively in silage fermented at low temperature.

## Claims

1. A lactic acid bacterium for preparing silage, constituted by a Lactobacillus diolivorans having anti-yeast action, wherein the Lactobacillus diolivorans is the Lactobacillus diolivorans SBS-0006 strain (NITE BP-02208), Lactobacillus diolivorans SBS-0007 strain (NITE BP-02209), or Lactobacillus diolivorans SBS-0009 strain (NITE BP-02210).

2. Silage comprising the lactic acid bacterium Lactobacillus diolivorans according to claim 1.

3. An additive for preparing silage, which comprises the lactic acid bacterium Lactobacillus diolivorans according to claim 1.

4. A method for preparing silage, **characterized by** adding the additive for preparing silage according to claim 3 to a silage material.

## Patentansprüche

1. Milchsäurebakterium zum Herstellen von Silage, bestehend aus einem Lactobacillus diolivorans mit Anti-Hefe-Wirkung,
wobei der Lactobacillus diolivorans der Lactobacillus diolivorans Stamm SBS-0006 (NITE BP-02208), der Lactobacillus diolivorans Stamm SBS-0007 (NITE BP-02209) oder der Lactobacillus diolivorans Stamm SBS-0009-(NITE BP-02210) ist.

2. Silage, die das Milchsäurebakterium Lactobacillus diolivorans nach Anspruch 1 umfasst.

3. Zusatzstoff zum Herstellen von Silage, der das Milchsäurebakterium Lactobacillus diolivorans nach Anspruch 1 umfasst.

4. Verfahren zum Herstellen von Silage, **gekennzeichnet durch** Zugeben des Zusatzstoffs zum Herstellen von Silage nach Anspruch 3 zu einem Silagematerial.

## Revendications

1. Bactérie lactique destinée à la préparation d'ensilage, constituée par un *Lactobacillus diolivorans* présentant une activité anti-levure, le *Lactobacillus diolivorans* étant la souche SBS-0006 (NITE BP-02208) de *Lactobacillus diolivorans,* la souche SBS-0007 (NITE BP-02209) de *Lactobacillus diolivorans,* ou la souche SBS-0009 (NITE BP-02210) de *Lactobacillus diolivorans.*

2. Ensilage comprenant la bactérie lactique *Lactobacillus diolivorans* selon la revendication 1.

3. Additif de préparation d'ensilage, qui comprend la bactérie lactique *Lactobacillus diolivorans* selon la revendication 1.

4. Procédé de préparation d'ensilage, **caractérisé par** l'ajout de l'additif de préparation d'ensilage selon la revendication 3 à un matériau d'ensilage.
